# EUROPEAN PATENT SPECIFICATION

(11) **EP 1 129 069 B1**
(45) Date of publication and mention of the grant of the patent: **10.09.2003**
(21) Application number: 99914791.1
(22) Date of filing: 16.04.1999
(51) Int. Cl.: C07C 237/46, C07C 231/24

(54) **METHOD FOR THE CRYSTALLIZATION OF IOPAMIDOL**
VERFAHREN ZUR KRISTALLISATION VON IOPAMIDOL
PROCEDE DE CRISTALLISATION DU IOPAMIDOL

(30) Priority: 09.11.1998 KR 9847785
(43) Date of publication of application: 05.09.2001
(73) Proprietor: DONG KOOK PHARMACEUTICAL CO., LTD., Kangnam-gu, Seoul 135-280 (KR)
(72) Inventor: PARK, Jin Kyu, Kangdong-ku, Seoul (KR); CHOI, Kyung Seok, Icheon city (KR); LEE, Dong Yub, Heungdeok-ku, Cheongju city (KR); LEE, In Kyu, Jincheon-kun, Chungcheongbuk-do (KR); SONG, Chang Hun, Heungdeok-ku, Cheongju city (KR); LEE, Dong Jun, Jecheon city (KR)
(74) Representative: Schweitzer, Klaus, Dr.
(86) International application number: KR9900181
(87) International publication number: WO00027804

(56) References cited:
- EP-A1- 0 747 344
- WO-A1-98/34908
- US-A- 5 571 941
- US-A- 5 689 002

## Description

### Technical Field

The present invention relates to a novel method for crystallizing Iopamidol to an anhydrous form, which is generally used as an X-ray contrast medium. More particularly, the present invention is concerned with a method for purifying Iopamidol to pharmaceutically acceptable purity, by which anhydrous Iopamidol can be produced at industrial yields.

### Background Art

Crystalline forms of Iopamidol exist as an anhydrous, monohydrated or pentahydrated state. Of them, anhydrous Iopamidol is pharmaceutically acceptable and thus, is now produced industrially. Techniques with which industrially beneficial Iopamidol can be obtained are found in U.S. Pat. Nos. 5,571,941 and 5,689,002.

U.S. Pat. No. 5,571,941 discloses a method for purifying Iopamidol, teaching that use of butanol, such as n-butanol, sec-butanol, isobutanol or t-butanol, as a crystallization solvent allows pharmaceutically pure Iopamidol with industrially acceptable yields. On the other hand, U.S. Pat. No. 5,689,002 discloses a method for crystallizing Iopamidol, in which water is used as a crystallization solvent to yield anhydrous pure Iopamidol which meets the pharmacopeia standards.

EP-A 0 747 344 discloses a process for the purification and crystallization of iopamidol which comprises adding 1-propanol or 2-propanol to an aqueous solution of iopamidol followed, if necessary, by azeotropic distillation. Example 5 of EP-A 0 747 344 discloses dissolving iopamidol in water heating to 68-72 °C, adding 2-propanol previously heated to 68-72 °C, refluxing for 60 minutes, cooling to 0-5 °C and filtering off the crystallized iopamidol.

### Disclosure of the Invention

The intensive and thorough research on the crystallization of Iopamidol, repeated by the present inventors, resulted in the surprising finding that a mixture of water and alcohol allows Iopamidol to be crystallized into an anhydrous pure form without producing monohydrates and pentahydrates.

As far as the present invention is concerned, Iopamidol, whether it exists as a monohydrate or a pentahydrate, can be crystallized into highly pure, anhydrous forms with industrially available yields.

Therefore, it is an object of the present invention to provide a method for the crystallization of Iopamidol, by which anhydrous Iopamidol with high purity can be industrially produced.

It is another object of the present invention to provide a method for purifying Iopamidol to pharmaceutically acceptable purity.

These and other objects can be attained in a method for crystallizing. Iopamidol, comprising the steps of dissolving Iopamidol in deionized water, distilling a part of the water off under a vacuum condition, adding alcohol to the aqueous solution, subjecting the solution to reflux for 3-4 hours to give white precipitates, filtering the precipitates, and drying the precipitates to yield pure anhydrous Iopamidol.

Particularly preferable is that the deionized water is used at an amount of water : Iopamidol from 1:10 to 1:1 (v/w) and the alcohol is used at an amount of alcohol : Iopamidol from 1:1 to 1:10 (v/w).

Further preferable is that the alcohol is selected from the group consisting of ethanol and propanol.

### Best Modes for Carrying Out the Invention

To be useful for injection, Iopamidol, which is used as an X-ray contrast medium, must exist as an anhydrous white crystalline form, but not as monohydrates or pentahydrates, and exhibit a solubility of 80% (w/v) in water. In addition, for industrial application, anhydrous Iopamidol must be produced with high purity at high yields while avoiding residual solvents which may cause an environmental problem.

In accordance with the present invention, a mixture of alcohol and water is used as a crystallization solvent for Iopamidol. The crystallization of the present invention starts by dissolving Iopamidol, whether existing as a monohydrate or a pentahydrate, in deionized water. 'Under a vacuum condition, this aqueous suspension is concentrated to distill off a part of the deionized water. The concentrated mixture is added with alcohol and brought to reflux for a predetermined period of time. During the reflux, Iopamidol begins to precipitate as white crystals. These precipitates are filtered off and dried.

Useful in the present invention is ethanol or propanol. This alcohol is used at an amount of alcohol : Iopamidol from 1:1 to 1:10 (v/w). As for water, it is used at an amount of water : Iopamidol from 1:10 to 1:1 (v/w).

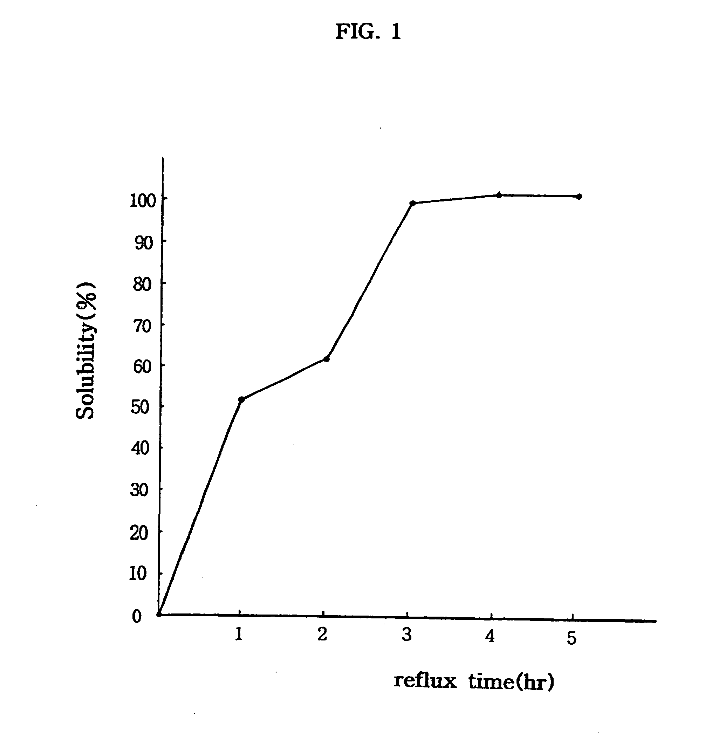

With reference to Fig. 1, there is a graph showing the solubility of Iopamidol plotted against reflux time when a ratio of Iopamidol : water : ethanol 4:1:8 (w/v/v) is used in accordance with the method of the present invention. As shown in this graph, the reflux for 3 hours or greater allows the crystallized Iopamidol to have a solubility of 80% or greater. This indicates that, when the reflux continues to be performed for 4 hours, only pure anhydrous Iopamidol exists. Because Iopamidol monohydrates or pentahydrates are of about 50% solubility, the method of the present invention can convert Iopamidol monohydrates or pentahydrates into Iopamidol anhydrides.

A better understanding of the present invention may be obtained in light of the following examples which are set forth to illustrate, but are not to be construed to limit the present invention.

### EXAMPLE I

10 kg of Iopamidol was dissolved in 10 liters of deionized water, followed by vacuum distilling 8 liters of the water. After being added with 5 liters of ethanol, the suspension was subjected to reflux for 4 hours. During the reflux, Iopamidol began to precipitate as white crystals which were, then, filtered off. After drying at 60 °C for 4 hours in vacuo, 7.5 kg of pure anhydrous Iopamidol was obtained. Yield : 75%.

### EXAMPLE II

10 kg of Iopamidol was dissolved in 10 liters of deionized water, followed by vacuum distilling 7.5 liters of the water. After being added with 20 liters of ethanol, the suspension was subjected to reflux for 4 hours. During the reflux, Iopamidol began to precipitate as white crystals which were, then, filtered off. After drying at 60 °C for 4 hours in vacuo, 9.3 kg of pure anhydrous Iopamidol was obtained. Yield : 93%.

### EXAMPLE III

10 kg of Iopamidol was dissolved in 10 liters of deionized water, followed by vacuum distilling 8 liters of the water. After being added with 20 liters of propanol, the suspension was subjected to reflux for 4 hours. During the reflux, Iopamidol began to precipitate as white crystals which were, then, filtered off. After drying at 60 °C for 4 hours in vacuo, 9.41 kg of pure anhydrous Iopamidol was obtained. Yield : 94.1%.

### EXAMPLE IV

10 kg of Iopamidol was dissolved in 10 liters of deionized water, followed by vacuum distilling 8 liters of the water. After being added with 30 liters of ethanol, the suspension was subjected to reflux for 4 hours. During the reflux, Iopamidol began to precipitate as white crystals which were, then, filtered off. After drying at 60 °C for 4 hours in vacuo, 9.0 kg of pure anhydrous Iopamidol was obtained. Yield : 90%.

### EXAMPLE V

10 kg of Iopamidol was dissolved in 10 liters of deionized water, followed by vacuum distilling 7.5 liters of the water. After being added with 20 liters of propanol, the suspension was subjected to reflux for 4 hours. During the reflux, Iopamidol began to precipitate as white crystals which were, then, filtered off. After drying at 60 °C for 4 hours in vacuo, 9.4 kg of pure anhydrous Iopamidol was obtained. Yield : 94%.

### TEST EXAMPLE I

In order to determine proper reflux time, the same procedures of the above examples were repeated, except that the reflux was performed for 1, 2, 3, 4 and 5 hours per procedure round. The solubility of the Iopamidol obtained was measured by dissolving 80 g of the Iopamidol in 100 ml of water. The results are given in Table 1, below.

**TABLE 1**

| Solubility of Iopamidol according to Reflux Times | | | | | |
|---|---|---|---|---|---|
| Examples | Reflux Times (hour) | | | | |
| | 1 | 2 | 3 | 4 | 5 |
| Example I | 50 | 63 | 98 | 100 | 100 |
| Example II | 51 | 61 | 99 | 100 | 100 |
| Example III | 53 | 62 | 98 | 100 | 100 |
| Example IV | 51 | 63 | 99 | 100 | 100 |
| Example V | 52 | 62 | 100 | 100 | 100 |

### TEST EXAMPLE II

The purity of the anhydrous Iopamidol obtained in the above examples was measured using high performance liquid chromatography (HPLC). The results are given in Table 2, below.

**TABLE 2**

| Purity of Iopamidol | | | | | |
|---|---|---|---|---|---|
| | Examples | | | | |
| | I | II | III | IV | V |
| **Purity (Area %)** | 99.8 | 99.8 | 99.5 | 99.9 | 99.5 |

### Brief Description of the Drawing

Fig. 1 is a graph showing the solubility change of the Iopamidol with reflux time.

### Industrial Applicability

As described hereinbefore, the crystalizing method of Iopamidol, i according to the present invention, does not need active carbon for decolorization, in contrast to the conventional crystallizing technique from water, and nor causes harm to the human body owing to residual solvents which may be problematic in the conventional technique using butanol as a crystallization solvent. In addition, the method of the present invention allows anhydrous Iopamidol 99.5% or higher purity, with a yield of 90%, so that it can be industrially applied for the mass production of Iopamidol.

## Claims

1. A method for crystallizing Iopamidol, comprising the steps of:
dissolving Iopamidol in deionized water;
distilling a part of the water off under a vacuum condition;
adding alcohol to the aqueous solution;
subjecting the solution to reflux for 3-4 hours to give white precipitates;
filtering the precipitates; and
drying the precipitates to yield pure anhydrous Iopamidol.

2. A method as set forth in claim 1, wherein the deionized water is used at an amount of water : Iopamidol from 1:10 to 1:1 (v/w) and the alcohol is used at an amount of alcohol : Iopamidol from 1:1 to 1:10 (v/w).

3. A method as set forth in claim 1 or 2, wherein the alcohol is selected from the group consisting of ethanol and propanol.

## Patentansprüche

1. Ein Verfahren zur Kristallisierung von lopamidol, umfassend die folgenden Schritte:
Auflösen des lopamidols in entionisiertem Wasser;
Abdestillieren eines Teils des Wassers unter Vakuumbedingungen;
Hinzufügen von Alkohol zu der wässrigen Lösung;
Kochen der Lösung unter Rückflussbedingungen für 3-4 Stunden, wobei ein weißes Präzipitat anfällt;
Filtrieren des Präzipitats; und
Trocknen des Präzipitats, wobei reines wasserfreies lopamidol erhalten wird.

2. Verfahren nach Anspruch 1, wobei das entionisierte Wasser in einem Wasser zu lopamidol-Verhältnis von 1:10 bis 1:1 (Vol./Gew.) verwendet wird und der Alkohol in einem Alkohol zu lopamidol-Verhältnis von 1:1 bis 1:10 (Vol./Gew.) verwendet wird.

3. Verfahren nach Anspruch 1 oder 2, wobei der Alkohol ausgewählt wird aus der Gruppe bestehend aus Ethanol und Propanol.

## Revendications

1. Procédé pour cristalliser lopamidol, comprenant les pas suivants:
résoudre le lopamidol dans l'eau déionizé;
ajouter du alcool à la solution aqueuse;
traiter la solution sous reflux pour 3-4 heures pour obtenir des precipitats blancs;
filtrer les precipitats; et
secher les precipitats pour obtenir du pur lopamidol déhydratisé.

2. Procédé selon la revendication 1, dans laquelle l'eau déionizé est utiliser dans une relation eau : lopamidol de 1:10 à 1:1 (vol./poids) et l'alcool est utiliser dans une relation alcool : lopamidol de 1:1 à 1:10 (vol./poids).

3. Procédé selon les revendications 1 ou 2, dans laquelle l'alcool est choisi parmi l'éthanol et le propanol.
